# FASCICULE DE BREVET EUROPEEN

(11) **EP 3 022 288 B1**
(45) Date de publication et mention de la délivrance du brevet: **17.01.2018**
(21) Numéro de dépôt: 14758590.5
(22) Date de dépôt: 22.07.2014
(51) Int. Cl.: C12N 1/04, C05F 11/08, C12N 1/14, C12N 1/20, C12N 1/22, C12N 1/02, C12N 11/10, C12N 11/12

(54) **PROCEDE D'OBTENTION D'INOCULUM SOLIDE SUPPORTE DANS UN AGROGRANULAT SUPERABSORBANT A VOLUME VARIABLE FONCTIONNANT EN MINIFERMENTEUR NATUREL.**
HERSTELLUNGSVERFAHREN FÜR EINE FESTES INOKULUM IN EINEM SUPERABSORBIERENDEN LANDWIRTSCHAFTLICHEN PELLET MIT VARIABLEM VOLUMEN ZUM BETRIEB IN EINEM NATÜRLICHEN MINI-FERMENTER.
METHOD TO PRODUCE A SOLID INOCULUM CARRIED IN A SUPERABSORBENT AGRO-PELLET WITH A VARIABLE VOLUME, OPERATING IN A NATURAL MINI FERMENTER.

(30) Priorité: 19.07.2013 FR 1301727
(43) Date de publication de la demande: 25.05.2016
(73) Titulaire: AB7 Innovation S.A.S.U., 31450 Deyme (FR)
(72) Inventeur: CHELLE, René, F-31450 Deyme (FR); MAKOUMBOU, Urbain, F-31270 Frouzins (FR)
(86) Numéro de dépôt international: PCT/FR2014/000173
(87) Numéro de publication internationale: WO 2015/007964

(56) Documents cités:
- FR-A1- 2 940 297
- FR-A1- 2 963 361
- FR-A1- 2 990 943
- DAULATRAM B LUND ET AL: "Survival of Rhizobia in Nilgiris peat", CURRENT SCIENCE , vol. 42, no. 12 20 juin 1973 (1973-06-20), pages 412-415, XP055113270, Extrait de l'Internet: URL:http://www.currentscience.ac.in/Downlo ads/article_id_042_12_0412_0415_0.pdf [extrait le 2014-04-09]
- B. MILICIC, D. KUZMANOVIC, D. JOSIC, D. DELIC, N. RASULIC: "Carrier formulations and their effect on rhizobial growth", ROUMANIAN BIOTECHNOLOGICAL LETTERS, vol. 11, no. 3, 2006, pages 2713-2721, XP008168783,
- SIGURBJORN EINARSSON ET AL: "Production of Rhizobium Inoculants for Lupinus nootkatensis on Nutrient-Supplemented Pumice", APPLIED AND ENVIRONMENTAL MICROBIOLOGY, vol. 59, no. 11, 1 novembre 1993 (1993-11-01), pages 3666-3668, XP055112778,
- DATE R. A.: "Legume inoculant production", PROCEEDINGS OF THE INDIAN NATIONAL SCIENCE ACADEMY , vol. 40B, no. 6 1976, pages 667-686, XP002723286, Extrait de l'Internet: URL:http://www.new1.dli.ernet.in/data1/upl oad/insa/INSA_1/20005b19_667.pdf [extrait le 2014-04-09]

## Description

La présente invention se rapporte au domaine de la production d'auxiliaires technologiques biotiques stimulateurs utilisables en agriculture et en agronomie, en substitution partielle ou totale d'engrais et/ou de traitement phytosanitaire.

Il est connu que les inocula sont des bactéries ou des champignons microscopiques apportés dans le sol afin d'améliorer le développement des plantes par :
∘ libération dans le sol de facteurs nutritifs qui sont utilisés par la plante,
∘ établissement de relations symbiotiques avec le système racinaire de la plante pour permettre l'accomplissement de fonctions vitales à la plante, comme la fixation et l'utilisation de l'azote atmosphérique,
∘ action antagoniste contre les agents pathogènes de la plante.

A cet effet, les rhizobactéries qui nodulent les racines des légumineuses sont les plus couramment utilisées, tout comme les champignons mycorhiziens qui colonisent les racines pour aider la plupart des plantes à retenir l'eau pour une meilleure absorption des nutriments. Peuvent être utilisés en inoculation les microorganismes non pathogènes dont, sous appellation générique et sans être exhaustif, on cite par exemple : *Azotobacter* spp., *Acidovorax facilis, Bacillus subtilis, Flavobacterium* spp., *Pseudomonas* spp., *Rhodococcus rhodochrous, Bacillus chitinoporus, Bacillus laterosporus,* ainsi que d'autres genres. Pour la protection de la plante on peut également citer des microorganismes à effet préventif, sans que la liste soit limitative : *Bacillus thuringiensis, Saccharopolyspora spinosa, Meterhizium anisopliae* ou encore *Beauvaria bassiana.*

Les inocula commerciaux sont disponibles sous forme de poudres, de granulés et de liquides. En règle générale, la tourbe constitue le support préféré sous forme de poudre. Des supports minéraux, la kaolinite et la vermiculite, sont également utilisés, mais de façon plus restreinte. Les inocula sont parfois directement pelliculés sur les graines

La demande de brevet international WO 2004/112462 (29.12.2004) divulgue un support élaboré à partir de noir de carbone d'os animal formant des granulés allant de 0,001 mm à 10 mm, utilisable pour apporter du phosphore naturel, pour un contrôle biologique des pathogènes du sol, ainsi que pour la décontamination du sol et pour l'activation et la fertilisation du sol. Ce document cite cependant le brevet WO 96/37433 qui cible un désavantage de ce type de support dû à son mode d'obtention qui nécessite un traitement par des solutions acides et alcalines qui ne sont pas souhaitables pour l'immobilisation et la conservation des inocula microbiens d'un point de vue physico-chimique et biologique. Un autre désavantage de ce type de matériau est sa grande solubilité dans l'eau, ce qui abaisse fortement l'apport biologique à la culture. Ce type de support va permettre d'accélérer le développement microbien sans pour autant prolonger la durée de la phase de reproduction de la souche. C'est d'ailleurs le même résultat atteint et décrit dans le brevet US 5173424 qui utilise à cet effet l'ajout de lécithine de soja sur le support d'inoculum pour accélérer le développement de *Rhizobium japonicum*.

Plusieurs types de supports d'inocula solides ont été étudiés comme alternative à la tourbe, dont l'encapsulation de microorganismes dans des microparticules en matrice polymère permettant d'immobiliser lesdits microorganismes et qui apportent une facilité de production, de stockage et de manipulation (Cassidy M.B., Lee H. et Trevors J.T. 1996 - J. Ind. Microbiol. 16 :79-101). Le brevet français FR2590904 (4/12/1985) décrit l'incorporation de microorganismes filamenteux dans des billes d'alginate de calcium qui sont dispersées dans le sol sous forme gel obtenue après réhydratation des billes avec un tampon, par exemple un tampon phosphate. La demande de brevet WO 00/59949 (06/04/1999) décrit quant à elle un antifongique associé à une matrice chitosan. La chitine est également décrite comme support de biofertilisation bactérienne par la demande de brevet français FR 2 941 589 A1 (03/02/2009). Quoique de grande efficacité, la chitine présente un double problème : son coût trop élevé et sa disponibilité plutôt aléatoire et restreinte à une zone géographique limitée.

Par ailleurs, des supports d'origine végétale ont déjà été évoqués (Smith R.S 1992 - J. Microbiol. 25 :739-745). Cette notion est confirmée par les demandes de brevet FR 2 593 191 (1996) et EP 0236 156 A2 (17/01/1986) qui décrivent un support d'inoculum en pulpe de raisins séchée et broyée. Le chardon aussi est utilisé par certains auteurs.

Pour leur utilisation, ces supports subissent une série d'opérations à savoir :
- séchage dans une étuve à 70°C pendant 48 heures,
- broyage en pellets ou en poudre,
- neutralisation à un pH ajusté entre 6,8 et 7 par addition de CaCO₃.

Mais tous ces supports d'inoculum se limitent particulièrement au transport et au stockage des cellules microbiennes, sans en assurer une multiplication continue, quand bien même des éléments accélérateurs de développement y sont incorporés avec les microorganismes pour faciliter le redémarrage du cycle de développement une fois les conditions favorables réunies dans le sol. En effet, le pouvoir d'absorption d'eau de ces supports est relativement limité à un taux en poids d'eau de leur poids de : 17,5% pour la tourbe, 23,5% pour la tourbe de France, 17,8% pour la kaolinite, 19,25% pour la vermiculite et 26,25% pour le chardon. D'après Saint-Macary et Neyra (1992), le volume nécessaire optimal d'inoculum liquide pouvant être absorbé dans le support est généralement compris entre 60% et 70% du volume de rétention d'eau, ce qui limite d'avantage la capacité des supports qui ont été cités.

D'après les travaux de Stephens et Rask (2000), il est noté que le support doit présenter deux propriétés fondamentales : permettre la croissance des microorganismes en place et les maintenir sur une période acceptable de conservation. La propriété de multiplication des microorganismes n'est pas recherchée.

Le brevet européen EP 0 443 040 B1 (05/9/1990) décrit un autre type de dispositif de culture de microorganismes qui comprend un textile non-tissé recouvert ou imprégné d'éléments nutritifs en solution dans un milieu de culture contenant des microorganismes infectieux envers la vermine des plantes. Le textile ainsi imprégné est séché puis enroulé en plusieurs couches autour de la tige, de la branche ou du tronc d'arbre à traiter. Ce dispositif exclusivement externe reste tributaire des aléas climatiques qui vont avoir un effet direct sur la culture microbienne. Les microorganismes cultivés ne nécessitent aucune activité symbiotique avec la plante traitée.

Dans le même cadre, le brevet US 5786188 (28/7/1998) décrit la préparation d'un inoculum de champignons microscopiques. Ledit inoculum est constitué par un substrat fibreux d'origine végétale, en pellets enrobés avec une suspension de champignons microscopiques dans de l'hydrogel, un alginate ou de l'acide alginique. Les éléments nutritifs sont dans les pellets, mais pour un développement des microorganismes en surface, exclusivement des champignons. Ce type de support spécifique aux champignons microscopiques ne peut servir à la culture de bactéries qui se développent en milieu fermé ou semi-fermé.

Il existe donc, au regard de l'art antérieur, le besoin de fournir un procédé d'obtention d'inoculum solide dont le support offre au moins les fonctions fondamentales sous-citées, en étant à la fois :
- support de stockage et de conservation de microorganismes revivifiables,
- support de transport de microorganismes dans les meilleures conditions de manipulation,
- support de croissance des microorganismes pour leur revivification et
- support de multiplication des microorganismes revivifiés,
- support de protection des microorganismes face aux éléments du milieu interstitiel,
- support de protection des microorganismes et de leur milieu de culture contre les effets de lessivage,
- support de protection des microorganismes contre le stress hydrique.

Lesdites fonctions permettent avantageusement d'obtenir une densité importante et durable de microorganismes dans le milieu interstitiel, pour optimiser par exemple les relations symbiotiques entre le système racinaire et les microorganismes ou l'impact biotique stimulant le développement et la productivité de la plante ou protégeant cette dernière.

Pour mieux comprendre l'invention, on entend par **« *agrogranulat superabsorbant à volume variable* »**, au singulier ou au pluriel, un granulé obtenu par le traitement chimique et thermomécanique à l'aide d'un dispositif d'extrusion muni de deux vis sans fin corotatives assurant la plastification de la matière par malaxage sous cisaillement et mise en pression de polymères d'origine végétale et ayant une grande capacité d'absorption d'eau ou de solutions aqueuses en augmentant de volume sans se déliter, puis revenant à son volume initial en se déshydratant.

<Au cours de la fabrication de l'agrogranulat dans un dispositif d'extrusion, une solution aqueuse de sel de sulfites est rajoutée dans le mélange solide constitué de biopolymères et de tissus naturels de cellules hyaline ou hydrocystes de manière à obtenir un mélange comprenant 10 à 45 % d'eau.>

On entend par **« *minifermenteur naturel* »**, au singulier ou au pluriel, un agrogranulat superabsorbant à volume variable dans lequel est stocké du milieu nutritif ou milieu de culture anhydre dont vont profiter les cellules microbiennes revivifiables introduites dans ledit agrogranulat pour y croître puis s'y multiplier lorsque les conditions hydriques sont satisfaites par un apport d'eau. Les microorganismes produits peuvent sortir par migration via le réseau poreux dudit agrogranulat.

On entend par **« *minifermenteur naturel continu* »**, au singulier ou au pluriel, le minifermenteur précédemment défini dans lequel un apport d'eau répété solubilise le milieu nutritif ou milieu de culture de manière continue et permet ainsi sa disponibilité renouvelée pour les microorganismes qui peuvent ainsi continuer à se multiplier. Il est entendu que lesdits microorganismes, au fur et à mesure de leur production, migrent dudit minifermenteur dans le milieu interstitiel et vers le système racinaire pour être pris en compte par la relation symbiotique avec les plantes.

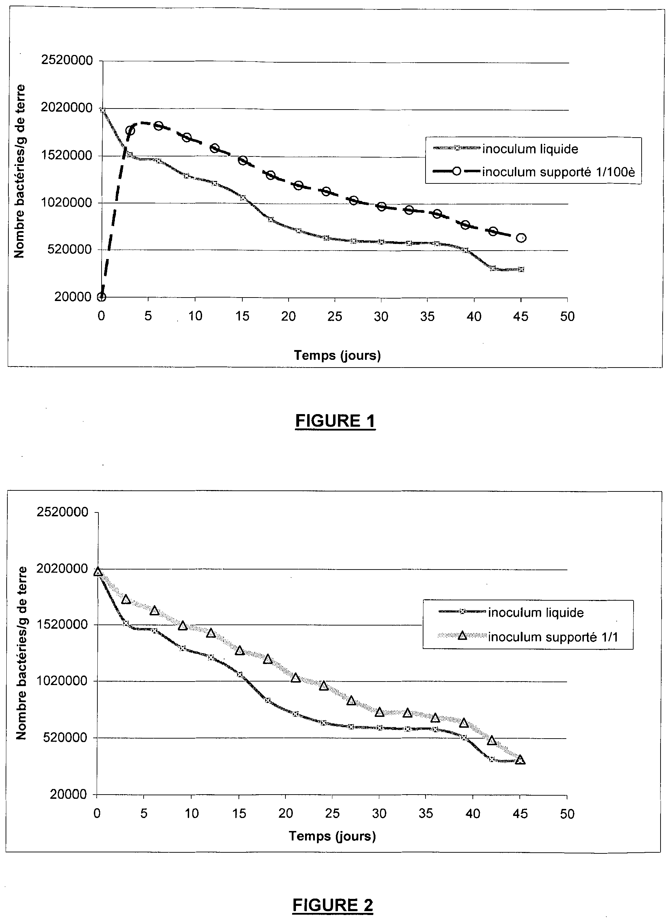

On entend par **« *milieu interstitiel* »** l'espace dans le sol entre l'inoculum solide et le système racinaire de la plante.
**La** **figure 1** représente les courbes de l'évolution comparée de deux inocula : inoculum liquide et inoculum supporté à 1/100^{e}
**La** **figure 2** représente les courbes de l'évolution comparée de deux inocula : inoculum liquide et inoculum supporté à 1/1

La demande de brevet FR 2990943 (22/05/2012), déposée par la demanderesse de la présente, décrit un agrogranulat superabsorbant à volume variable présentant la particularité de pouvoir absorber et mettre en réserve de l'eau ou une solution aqueuse en grande quantité allant jusqu'à 500% en poids sans se déliter et sans percolation.

On propose de surmonter les inconvénients des systèmes d'inocula antérieurs évoqués précédemment grâce à un agrogranulat superabsorbant à volume variable fonctionnant en minifermenteur naturel pouvant absorber une substance ou des éléments microscopiques en suspension hydrique sans les perdre par lessivage. On souhaite en particulier pouvoir fabriquer un système d'inoculum microbien solide supporté dans ledit agrogranulat, réalisant en même temps la fonction de support et la fonction de fermenteur permettant la multiplication en son sein des microorganismes composant ledit inoculum et dont les divers nutriments et facteurs de croissance du milieu nutritif se trouvent déjà à l'état sec dans ledit fermenteur sans risque d'être lessivés. Ledit système d'inoculum est ainsi conditionné pour accroître son efficacité et sa durée d'activité biologique.

On propose la réalisation d'un nouveau type d'inoculum solide supporté en quantité conséquente dans un support biodégradable avec son milieu nutritif en quantité nécessaire et également sec, dont les cellules microbiennes peuvent revivifier puis croître et se multiplier comme dans un fermenteur, dès lors que le support est réhydraté, y compris lorsque ledit support est enterré. Le choix des microorganismes à valeur d'usage fixée composant l'inoculum doit tenir compte du fait que ceux-ci soient en capacité de pouvoir se multiplier même en milieu fermé et de pouvoir migrer hors du minifermenteur grâce à une caractéristique biologique d'affinité avec le système racinaire de la plante.

L'inoculum solide est supporté dans un agrogranulat superabsorbant à volume variable fonctionnant en minifermenteur naturel, préchargé en milieu nutritif anhydre favorisant la croissance suivie de la multiplication continue des cellules microbiennes revivifiées, par réhydratation et dissolution progressives dudit milieu nutritif, en évitant son lessivage par les eaux de pluie et d'arrosage et en stabilisant ainsi cette source biologique pour accroître son efficacité et sa durée d'activité biotique.

L'inoculum solide est supporté dans un agrogranulat superabsorbant à volume variable fonctionnant en minifermenteur naturel continu préchargé en milieu nutritif concentré anhydre favorisant la multiplication continue des microorganismes revivifiés incorporés en faible densité, pour une efficacité et une durée d'activité biologique accrues, ledit inoculum assurant les propriétés fondamentales permettant :
a. la conservation et le stockage des microorganismes revivifiables,
b. le transport des microorganismes dans les meilleures conditions de manipulation et d'application en champs,
c. la croissance des microorganismes revivifiés pour une reprise du cycle de développement lorsque les conditions favorables sont réunies,
d. la multiplication intra-support des microorganismes,
e. la protection des microorganismes face aux éléments négatifs du milieu interstitiel,
f. la protection des microorganismes et de leur milieu de culture contre les effets de lessivage,
g. la protection des microorganismes contre le stress hydrique.

Ledit support est un agrogranulat superabsorbant à volume variable obtenu selon le procédé décrit par la demande de brevet FR 2990943. Il apporte un gain sur le procédé technologique dans la mesure où il ne nécessite pas les opérations décrites pour la mise en condition des supports déjà connus décrits dans l'art antérieur. En effet, il est à noter que :
- son pH par exemple est déjà ajusté lors de son élaboration,
- il est de granulométrie régulière favorable à la manipulation et à l'application en champs à l'aide des outillages couramment utilisés en agriculture,
- sa structure plastique lui confère la caractéristique de varier de volume en gonflement par absorption d'eau ou de solution aqueuse et en rétractation par perte ou désorption de ces dits liquides, permet d'éviter son délitement dans ces conditions,
- la capacité de varier son volume en absorbant de l'eau ou une solution aqueuse est favorable à la revivification rapide et à la multiplication des microorganismes dans le réseau poreux, contrairement à ce qui se passe dans matériaux poreux rigides dans lesquels les microorganismes étouffent,
- sa grande capacité d'absorption d'eau ou de solution aqueuse associée à son fort pouvoir de rétention d'eau ou de solution aqueuse permet d'éviter la percolation des liquides retenus et donc le lessivage des éléments en suspension ou dissous, tout en préservant les microorganismes du stress hydrique grâce à une présence d'eau toujours suffisante et non excédentaire dans ledit support,
- son grand réseau de pores favorise la migration des microorganismes de l'intérieur vers l'extérieur par différence de pression ou par les effets des agents biotiques.

Le procédé d'obtention dudit agrogranulat superabsorbant à volume variable selon FR 2990943 consiste à élaborer une matrice composite plastique biodégradable composée d'un mélange de biopolymères protéiques d'origine végétale (exemple : tourteaux de tournesol, de soja, de colza, de luzerne ou de lin), de biopolymères saccharidiques riches en amidon, hémicelluloses et pectines, et de tissus naturels de cellules hyalines ou hydrocystes (exemple : sphaigne).

Plus précisément, le support d'inoculum solide présente un taux de matière sèche compris entre 92% et 95%, ainsi qu'une grande capacité d'absorber et de mettre en réserve de l'eau ou une solution aqueuse, grâce à un pouvoir absorbant allant jusqu'à 500% en poids, permettant ainsi une activité biologique interne intense, ce qui lui confère la qualité de minifermenteur. Ledit support n'absorbe plus lorsque sa limite d'absorption est atteinte, ce qui préserve de la percolation et donc du lessivage car l'eau ou la solution aqueuse absorbée est gardée dans la structure.

L'élaboration dudit agrogranulat en minifermenteur naturel est réalisée en préchargeant l'agrogranulat respirant superabsorbant en milieu nutritif concentré liquide correspondant au genre de microorganismes à inoculer, puis en le déshydratant avant d'y incorporer lesdits microorganismes en phase liquide, puis de le déshydrater à nouveau avant de l'emballer pour stockage.

L'agrogranulat présente une matrice élaborée par plastification de matériaux naturels à l'aide d'un dispositif d'extrusion bivis et comprenant :
**a).** un mélange de biopolymères constitué de :
   **i.** 55% à 60% en poids de biopolymères protéiques riches en globulines, albumines, prolamines et également riches en fibres, choisis parmi les sous-produits de matières végétales protéagineuses (tournesol, soja, colza, luzerne, lin) et
   **ii.** 40% à 45% en poids de biopolymères polysaccharidiques riches en amidon, hémicelluloses et pectines, choisis parmi les farines de graines de céréales ou de la pulpe de betterave sucrière.
**b).** 5% à 40% en poids du mélange solide de tissus naturels de cellules hyalines ou hydrocystes, choisis parmi des matières végétales vivantes : sphaigne ou chardon *Carduus*

Selon une composition préférée qui conduit aux meilleurs résultats, le milieu nutritif est adapté à la souche de microorganismes à inoculer et formulé suivant une composition choisie optionnellement parmi :
a) YMB (Yeast - Mannitol - Broth) (Vincent, 1970),
b) YM (Yeast - Mannitol),
c) BHI (Brain Heart Infusion),
mais aussi tout autre milieu adéquat en rapport avec les microorganismes à traiter.

Le milieu de culture incorporé dans le support minifermenteur est en phase aqueuse dont le taux de matière sèche, constituée par divers nutriments et facteurs de croissance particulièrement favorables à la souche de microorganismes à inoculer, est de 2 à 10 fois plus élevé que celui du milieu de culture liquide adapté selon la norme recommandée pour la même souche de microorganismes. Avantageusement, le volume de milieu nutritif liquide concentré à incorporer dans ledit support peut varier entre 25% et 500% en poids.

Le milieu nutritif concentré est caractérisé par le fait qu'à volume final égal, il contient 2 à 10 fois plus de nutriments et/ou d'éléments favorisant la croissance et la multiplication des microorganismes en culture que le milieu nutritif normal. Lesdits éléments et/ou nutriments sont bien connus de l'homme du métier en fonction des types de microorganismes en culture. A titre d'exemple, on peut citer l'extrait de levure, des sucres simples tels que le mannitol, le glucose ou le saccharose, des sels tels que le chlorure de sodium, le sulfate de magnésium, etc.

Les microorganismes concernés peuvent être, sans pour autant que cette liste soit limitative, des champignons mycorhiziens, des rhizobactéries, ainsi que d'autres microorganismes pouvant contribuer à l'amélioration de la vie des plantes, à leur protection et à leur traitement biotique comme : *Azotobacter* spp., *Acidovorax facilis, Flavobacterium* spp., *Pseudomonas* spp., *Rhodococcus rhodochrous, Bacillus subtilis, Bacillus chitinoporus, Bacillus laterosporus, Bacillus thuringiensis, Saccharopolyspora spinosa, Meterhizium anisopliae* ou encore *Beauvaria bassiana*.

Selon un mode de fonctionnement, l'eau ou la solution aqueuse absorbée en petite quantité dans le minifermenteur ne dissout que partiellement le milieu de culture sec qui s'y trouve en forte concentration, en respectant les lois physico-chimiques de la dissolution, permettant ainsi aux cellules microbiennes revivifiables stockées de s'en servir pour croître et se multiplier. En effet, la présence du milieu nutritif permet une revivification rapide des cellules microbiennes. La dissolution partielle du milieu de culture anhydre permet d'assurer une réserve pour la continuité du cycle de production des cellules microbiennes dans le minifermenteur. La présence du milieu de culture à l'état sec dans le minifermenteur contribue par ailleurs à l'optimisation du volume d'eau stockée à l'avantage d'une activité hydrique favorable aux microorganismes. En effet, une activité hydrique trop élevée peut conduire à une mortalité élevée des cellules microbiennes.

Les cellules microbiennes produites dans le minifermenteur migrent par le réseau poreux dudit minifermenteur vers le milieu interstitiel et le système racinaire de la plante réceptrice, au fur et à mesure de leur production, grâce à la relation symbiotique et aux dispositions biologiques des deux entités cellules microbiennes et système racinaire récepteur. Le cycle de production des cellules microbiennes dans ledit minifermenteur peut ainsi continuer tant qu'il y a de l'eau apportée et du milieu nutritif dissous accessible aux microorganismes avec une activité hydrique adaptée, les métabolites produits par les cellules microbiennes à l'intérieur du minifermenteur étant exportés par la migration des cellules vivantes vers le milieu interstitiel. Toutefois, la quantité d'eau ou de solution aqueuse pouvant être absorbée par le support est limitée naturellement par la limite du pouvoir absorbant et aussi par la présence du milieu nutritif anhydre dans ledit agrogranulat.

Le minifermenteur mis en conditions de fonctionnement continu permet de réduire considérablement la densité de cellules microbiennes viables à inoculer dans ledit support à une densité comprise entre 1/500^{e} et 1/10^{e} de la quantité recommandée par la norme pour l'inoculum liquide, mais préférentiellement à une densité comprise entre 1/200^{e} et 1/100^{e} de ladite quantité recommandée par la norme pour l'inoculum liquide. Cette densité atteint rapidement la densité recommandée par la norme en quelques jours seulement. Pour un inoculum liquide, la recommandation préconise une densité de Rhizobium comprise entre 10⁶ et 10⁸ cellules/mL (Padmanabhan Somasegaran et Jake Halliday, Applied and Environmental Microbiology, vol. 44, 1982). Les normes indiquent par exemple un nombre de *Rhizobium* spp. par gramme de support d'au moins 5 x 10⁸ cellules (Beck et *al.*, 1993). En inoculant selon l'invention par exemple *n* x 10⁴ cellules par gramme (*n* variant entre 2 et 9) dans le minifermenteur, on obtient la densité de cellules viables recommandée en 3 à 5 jours dans le milieu interstitiel, ce qui représente un gain très important de volume de support et d'inoculum pour un coût opérationnel moindre par rapport aux rendements attendus en apport de facteurs de développement à la plante, ou de l'envergure de l'action antagoniste contre les agents phytopathogènes. La densité maximale recommandée de cellules viables pour un inoculum solide est de 10⁶ cellules/g de support (Fichier technique de la fixation symbiotique de l'azote - Organisation des Nations Unies pour l'Alimentation et l'Agriculture (FAO) - 1992).

En effet, de manière inattendue, le niveau de production de cellules microbiennes à partir du minifermenteur, en partant de *n x* 10⁴ cellules par gramme de support, atteint des valeurs comprises entre *n x* 10⁹ cellules et *n* x 10¹⁰ cellules par gramme de support en une durée variable, en fonction des séquences d'apport d'eau et de la souche microbienne inoculée, ce qui est largement supérieur à la norme conseillée selon la recommandation du manuel de la FAO qui est de *n x* 10⁸ cellules par gramme de support. On note une efficacité et une durée d'activité biologiques accrues exprimées par une bio-augmentation de la population microbienne qui peut ainsi se multiplier en intra-support de *n x* 10⁴ cellules à *n x* 10⁶ cellules supplémentaires pour atteindre en l'espace de 3 à 5 jours des densités de l'ordre de *n* x 10¹⁰ UFC (Unité Formant Colonie) dans le sol ensemencé avec le support inoculé.

Le support fonctionne donc en véritable fermenteur dans lequel les microorganismes croissent et se multiplient vite en partant d'une faible densité pour atteindre rapidement et voire dépasser les valeurs de densités microbiennes préconisées par les normes selon la recommandation du manuel de la FAO.

La capacité de rétention d'eau du support minifermenteur permet d'éviter le lessivage tant du milieu de culture que des cellules microbiennes par l'eau de pluie ou d'arrosage. L'inoculum supporté se maintient à proximité du système racinaire et augmente ainsi les rendements des relations symbiotiques entre le système racinaire de la plante et les cellules microbiennes devant le coloniser.

Selon l'invention, l'inoculum solide supporté dans un agrogranulat superabsorbant à volume variable fonctionnant en minifermenteur naturel est obtenu par la mise en oeuvre du procédé qui consiste à :
a) incorporer dans le support en agrogranulat superabsorbant à volume variable le milieu nutritif liquide concentré convenant à la souche à inoculer, à un taux compris entre 25% et 500% en poids, mais préférentiellement à un taux à valeur d'usage fixée adaptée aux microorganismes ;
b) déshydrater le support obtenu en a) entre 40°C et 55°C jusqu'à un taux de matière sèche compris entre 90% et 95% en poids ;
c) stériliser le support sec obtenu en b) dans des containers étanches ;
d) incorporer la souche microbienne ayant été préalablement cultivée dans le support stérile obtenu en c) en densité microbienne variant entre 1/500^{e} et 1/10^{e} de la quantité recommandée par les normes pour un inoculum liquide de la même souche, mais préférentiellement en densité microbienne comprise entre 1/200^{e} et 1/100^{e} de la quantité recommandée par les normes pour un inoculum liquide de la même souche ;
e) déshydrater le support chargé obtenu en d) par étuvage entre 35°C et 40°C pour obtenir un inoculum solide supporté à un taux de matière sèche compris entre 90% et 95% en poids ;
f) emballer l'inoculum solide supporté obtenu en e) à l'abri de l'humidité.

Selon le mode de mise en oeuvre du procédé de l'invention, l'incorporation du milieu nutritif liquide concentré ainsi que la biomasse à inoculer dans le support se fait préférentiellement par aspersion, mais elle peut être également faite par pulvérisation ou encore par trempage rapide.

Selon le mode de mise en oeuvre du procédé de l'invention, la stérilisation des agrogranulats se fait en autoclave, par irradiation ou encore par tyndallisation.

L'inoculum solide supporté dans un agrogranulat superabsorbant à volume variable fonctionnant en minifermenteur présente de nombreux avantages, le premier étant d'être pratique, de pouvoir être stocké et transporté en emballages hermétiques en toute quiétude, afin d'être appliqué facilement selon la disponibilité de l'agriculteur, en utilisant du matériel agricole courant. Il n'est pas emporté par le vent, ne sature pas les sols parce qu'il est biodégradable. Il permet d'éviter les effets de lessivage tant de la population microbienne utile au système racinaire naissant que du milieu de culture nécessaire à ladite population microbienne. Le milieu nutritif incorporé joue à la fois un rôle de starter pour le démarrage du cycle et de milieu de culture pour la multiplication intra-support continue de la souche de microorganismes inoculée. Il met à l'abri du stress hydrique la biomasse microbienne en développement en son sein.

L'utilisation de l'inoculum solide supporté en minifermenteur permet de minimiser la quantité d'inoculum à appliquer, ce qui est source d'économie conséquente dans la gestion de la production de la biomasse microbienne à partir d'une souche spécifique. Il permet également de protéger la souche microbienne contre les effets négatifs des éléments du sol comme son pH et sa composition élémentaire chimique qui d'ailleurs influence beaucoup le pH.

Les exemples qui suivent permettront d'illustrer l'invention et de mieux en comprendre les différents avantages. Par commodité, dans ce qui suit les abréviations suivantes seront utilisées :
- UFC : unité formant colonie
- YM : milieu nutritif liquide (Yeast Mannitol)
- YM2x : milieu nutritif liquide 2 fois plus concentré que le milieu liquide YM

### EXEMPLE 1 - Comparaison de l'évolution dans la terre d'un inoculum liquide et d'un inoculum chargé dans un support minifermenteur

On utilise un agrogranulat respirant superabsorbant en tourteau de tournesol et sphaigne fabriqué dans les laboratoires de la demanderesse, ayant une capacité d'absorption de 500% en poids de liquide.

La souche utilisée est une bactérie du sol du genre Rhizobium : *Rhizobium phaseoli* DSM 30137 de la collection DSMZ (deutsche Sammlung von Mikroorganismen und Zelkulturen)

On utilise un milieu de culture spécifique à cette souche dont la composition est décrite dans les tableaux 1 et 2. La concentration du milieu de culture (YM) est celle qui est normalement utilisée pour la culture de la souche en présence, tandis que la concentration du milieu de culture (YM2x) destiné à être incorporé dans le support est 2 fois la concentration normale de culture (YM).

**Tableau 1 : composition du milieu de culture liquide YM**

| **Produit** | **Fournisseur** | **Référence** | **Concentration** |
|---|---|---|---|
| Extrait de Levure | BIOCAR | A1202GC | 0,4 g/L |
| Mannitol | PROLABO | 25311.297 | 10,0 g/L |
| K₂HPO₄ | PROLABO | 26931.263 | 0,5 q/L |
| MgSO₄, 7H₂O | SIGMA | M1880 | 0,2 q/L |
| NaCl | PANREAC | 121659,1211 | 0,1 g/L |
| pH ajusté à 7,0 (H₂SO₄), stérilisation en autoclave 20 minutes à 121°C | | | |

**Tableau 2 : composition du milieu de culture liquide YM2x**

| **Produit** | **Fournisseur** | **Référence** | **Concentration** |
|---|---|---|---|
| Extrait de Levure | BIOCAR | A1202GC | 0,8 g/L |
| Mannitol | PROLABO | 25311.297 | 20,0 g/L |
| K₂HPO₄ | PROLABO | 26931.263 | 1,0 g/L |
| MgSO₄, 7H₂O | SIGMA | M1880 | 0,4 g/L |
| NaCl | PANREAC | 121659,1211 | 0,2 g/L |
| pH ajusté à 7,0 (H₂SO₄), stérilisation en autoclave 20 minutes à 121°C | | | |

On asperge 1 gramme d'agrogranulats avec 1 millilitre de milieu nutritif concentré YM2x pour son incorporation, soit une incorporation de 100% du poids d'agrogranulats en YM2x. Puis les agrogranulats sont séchés à 92% en poids de matière sèche.

Puis, on inocule 10⁶ bactéries soit 1/100^{è} de la quantité recommandée par les normes en milieu liquide, toujours par aspersion sur les agrogranulats secs pour obtenir un inoculum sur support.

On prépare des lots de 100 grammes de terre broyée et stérilisée que l'on dispose dans des boîtes de Pétri carrées de 1 dm² de superficie. On répartit les lots ainsi constitués en deux groupes :
- Groupe 1 dit ESSAI N°1 avec des lots de 100 g de terre inoculés avec 1 g d'inoculum sur support, environ 10⁶ bactéries/dm² soit 10⁴ bactéries/g de terre ;
- Groupe 2 dit ESSAI N°2 avec des lots de 100 g terre inoculés avec un inoculum liquide pour 10⁶ bactéries/dm² soit 10⁴ bactéries/g de terre.

On pulvérise 5 ml d'eau courante stérile à la surface de la terre et on met à incuber à 28°C. L'arrosage est répété à l'identique tous les jours.

Après 5 jours d'essai, on procède au dénombrement des bactéries dans chaque lot et on compare la moyenne de l'ESSAI N°1 avec celle de l'ESSAI N°2.

On obtient dans l'ESSAI N°1 un plateau d'environ 3,4.10¹⁰ UFC/boîte. En comparaison, on obtient dans l'ESSAI N°2 un plateau d'environ 1,5.10¹⁰ UFC/boîte, soit moins de la moitié de l'ESSAI N°1, ce qui donne une différence très importante de l'ordre de 1,9.10¹⁰ UFC/boîte.

Le support en agrogranulats respirants superabsorbants a donc fonctionné en fermenteur.

### EXEMPLE 2 - Etude comparé entre 3 inocula sur une longue durée : inocula liquide, un inoculum supporté avec 1/100^{e} de l'inoculum liquide et un inoculum supporté à 1/1 de l'inoculum liquide (Figures 1 et 2)

On utilise de la terre de culture prélevée dans un champ régulièrement cultivé. On utilise comme support d'inoculum des agrogranulats respirants superabsorbants fabriqués dans les laboratoires d'AB7 INNOVATION.

On utilise des bactéries du sol du genre Rhizobium de la souche *Rhizobium leguminosarum* CPI 106959 de l'Institut Pasteur.

On utilise un milieu de culture liquide YM, dont la composition est donnée par le tableau 3, pour la culture de la souche et comme constituant de l'inoculum liquide. Tandis que le milieu de culture YM2x est utilisé pour enrichir le support avant d'y inoculer des bactéries ; sa composition, donnée dans le tableau 4, indique qu'il est 2 fois plus concentré que le milieu YM.

**Tableau 3 : Composition du milieu de culture liquide YM**

| **Produit** | **Fournisseur** | **Référence** | **Concentration** |
|---|---|---|---|
| Extrait de levure | VWR | VM445253.224 | 1 g |
| Mannitol | VWR | K93281282.239 | 10 g |
| Extrait de terre | AB7 INNOVATION | ECH4158T | 200 ml |
| Eau distillée | AB7 INNOVATION | ECH4158E | 800 ml |
| pH ajusté à 7 ± 0,2 (H₂SO₄), stérilisation en autoclave 30 mn à 110°C | | | |

**Tableau 4 : Composition du milieu de culture liquide YM2x**

| **Produit** | **Fournisseur** | **Référence** | **Concentration** |
|---|---|---|---|
| Extrait de levure | VWR | VM445253.224 | 2 g |
| Mannitol | VWR | K93281282.239 | 20 g |
| Extrait de terre | AB7 INNOVATION | ECH4158T | 200 ml |
| Eau distillée | AB7 INNOVATION | ECH4158E | 800 ml |
| pH ajusté à 7± 0,2 (H₂SO₄), stérilisation en autoclave 30 mn à 110°C | | | |

### a. Préparation de la terre

De la terre de culture est placée dans un container étanche pour sa stérilisation en étuve à 110°C pendant 4 jours. Puis elle est répartie en 90 lots de 100 grammes.

### b. Montage des tests

On dispose de 90 pots stériles de 125 millilitres, de 6,5 centimètres de diamètre et 5,3 centimètres de hauteur, à fond percé de trous pour permettre le drainage à l'extérieur d'excès d'eau d'arrosage. Ils sont répartis en trois groupes de 30 pots : groupe 1 pour l'inoculum liquide, groupe 2 pour l'agrogranulat inoculé au 1/100^{e} de l'inoculum liquide et groupe 3 pour l'agrogranulat inoculé à 1/1 de l'inoculum liquide. On obtient une répartition de bactéries dans les pots précisée dans le tableau 5 :

**Tableau 5 : Répartition des bactéries dans les pots de terre**

| **Groupe de test** | **Inoculum** | **Nombre de bactéries/g de terre** |
|---|---|---|
| 1 | Inoculum liquide | 2.10⁶ |
| 2 | Agrogranulat à 1/100^{e} d'inoculum liquide | 2.10⁴ |
| 3 | Agrogranulat à 1/1 d'inoculum liquide | 2.10⁶ |

Puis on procède comme suit :
- Dans chaque pot, on place uniformément 80 grammes de terre stérile ;
- On y disperse uniformément l'inoculum à raison de 1 mL pour le liquide à 2.10⁸ cellules et 1g de support inoculé à 1/100^{e} soit 2.10⁶ cellules et à 1/1 soit 2.10⁸ cellules par pot de 100g de terre ;
- On recouvre avec le reste de terre soit 20 grammes, puis on tasse légèrement ;
- On arrose avec 15 cm³ d'eau distillée et on place les échantillons dans une étuve à 28°C.

### c. Suivi des tests

Pour chaque groupe, on prévoit un arrosage avec 15 cm³ d'eau tous les deux jours et 25 cm³ d'eau les derniers jours de semaine. On prélève deux pots de chaque groupe tous les quatre jours pour décomptage de cellules bactériennes. Pour cela :
- On mélange tout le contenu du pot pour une bonne homogénéisation dans un mixeur puis on en prélève 5g dans un tube à essai ;
- On verse 10ml d'eau stérile dans le tube puis on agite vigoureusement au vortex ;
- On laisse décanter puis on prélève le surnageant pour le comptage des UFC et l'observation et comptage au microscope. Les UFC sont réalisées sur gélose dans des boîtes de Pétri.

### d. Analyse des résultats

Les résultats obtenus après 45 jours de test sont illustrés par les graphiques des figures 1 et 2 qui représentent les courbes d'évolution des trois inocula : inoculum liquide, inoculum supporté à 1/100^{e} et inoculum supporté à 1/1. Leur analyse conduit aux conclusions suivantes :
1. La comparaison de l'évolution entre l'inoculum liquide et l'inoculum supporté à 1/1, illustrée par la figure 2, montre qu'en partant de la même densité de bactéries/gramme de terre, soit 2.10⁶, l'inoculum supporté résiste mieux, ce qui démontre que le support joue un rôle de protection de la population microbienne par rapport aux éléments négatifs du sol comme le pH par exemple, mais aussi les éléments environnementaux comme le lessivage par l'eau d'arrosage dont l'excès passe par les trous pratiqués dans le fond du pot.
2. L'observation de l'évolution de l'inoculum supporté à 1/100^{e} montre un effet de multiplication rapide des bactéries dans le support qui fonctionne ainsi en véritable fermenteur. Le milieu de culture sec contenu dans le support se dissout à l'arrosage pour jouer un grand effet starter et soutient par la suite la multiplication microbienne. L'inoculum supporté à 1/100^{e} finit ainsi par dépasser le niveau de l'inoculum liquide dès le 5^{ème} jour du test pour le rester jusqu'au dernier jour de l'étude (figure 1). On peut donc penser que le minifermenteur ainsi constitué joue également un rôle de protection de la population microbienne, mais aussi du milieu de culture dont les bactéries continuent à disposer pour se multiplier.
3. L'évolution comparée des deux inocula supporté, à 1/1 et 1/100^{e}, fait ressortir les phénomènes inhérents à la fermentation en fermenteur fermé à savoir :
   a. Une densité de cellules microbiennes trop élevée dans un fermenteur conduit à une mortalité de cellules due aux métabolites produits par la biomasse. Cette mortalité est atténuée par le renouvellement du milieu de culture. C'est le cas pour l'inoculum supporté dont le milieu de culture se renouvelle à chaque arrosage.
   b. La multiplication microbienne est plus probante en partant avec une plus faible densité de cellules pour le même volume de fermenteur. Avec 1/100^{e} de l'inoculum, on obtient des résultats meilleurs tout en réalisant une économie considérable sur la quantité d'inoculum à utiliser et un bénéfice conséquent sur les coûts.

En conclusion, l'utilisation du support minifermenteur naturel permet de protéger les microorganismes contre les éléments négatifs du sol et contre le lessivage tant du milieu de culture sans lequel il n'y aurait pas de continuité du cycle de production de cellules microbiennes de l'inoculum. Et de façon extraordinaire, le nombre de cellules microbiennes inoculées constituant l'inoculum de départ est réduit au 1/100^{e}, ce qui représente une économie conséquente en termes de temps et de coût de culture de la souche microbienne.

## Revendications

1. Procédé d'obtention d'un inoculum solide supporté constitué d'un agrogranulat, en matrice composite plastique biodégradable superabsorbant comprenant un mélange de biopolymères protéiques riches en globulines et en fibres, de biopolymères polysaccharidiques riches en amidon, hémicelluloses et pectines et de tissus naturels de cellules hyaline, ledit agrogranulat étant capable d'absorber de l'eau ou une solution aqueuse allant jusqu'à 500% de son poids, ledit agrogranulat étant préchargé en milieu nutritif ***caractérisé en ce qu'***il consiste à
a) incorporer dans le support en agrogranulat superabsorbant le milieu nutritif liquide concentré convenant à la souche à inoculer, à un taux compris entre 25% et 500% en poids, la concentration dudit milieu nutritif étant de 2 à 10 fois plus élevée que celle de milieu de culture liquide adapté ;
b) déshydrater le support obtenu en a) entre 40°C et 55°C jusqu'à un taux de matière sèche compris entre 90% et 95% en poids ;
c) stériliser le support sec obtenu en b) dans des containers étanches ;
d) incorporer la souche microbienne ayant été préalablement cultivée dans le support stérile obtenu en c) en densité microbienne variant entre 1/500^{e} et 1/10^{e} préférentiellement entre 1/200^{e} et 1/100^{e} de la quantité recommandée par les normes pour un inoculum liquide de la même souche ;
e) déshydrater le support chargé obtenu en d) par étuvage entre 35°C et 40°C pour obtenir un inoculum solide supporté à un taux de matière sèche compris entre 90% et 95% en poids ;
f) emballer l'inoculum solide supporté obtenu en e) à l'abri de l'humidité.

2. Procédé selon la revendication 1, ***caractérisé en ce que*** le milieu de culture anhydre concentré contenu dans le support se dissout partiellement et progressivement par l'eau de pluie ou d'arrosage sans risque d'être lessivé.

3. Procédé selon l'une des revendications 1 ou 2, ***caractérisé en ce que*** l'on réhydrate ledit agrogranulat pour la dissolution partielle et progressive dudit milieu nutritif afin de favoriser la croissance suivie de la multiplication continue des microorganismes revivifiés y compris lorsque ledit agrogranulat est enterré.

4. Procédé selon l'une des revendications 1 à 3, ***caractérisé en ce que*** ledit agrogranulat varie de volume en gonflement par absorption d'eau ou de solution aqueuse et en rétractation par relargage ces dits liquides, sans se déliter.

5. Procédé selon l'une des revendications 1 à 4, ***caractérisé en ce que*** les microorganismes sont choisis parmi les champignons mycorhiziens et les rhizobactéries.

6. Procédé selon la revendication 5, ***caractérisé en ce que*** les microorganismes sont choisis parmi *Azotobacter* spp., *Acidovorax facilis, Flavobacterium* spp., *Pseudomonas* spp., *Rhodococcus rhodochrous, Bacillus subtilis, Bacillus chitinoporus, Bacillus laterosporus, Bacillus thuringiensis, Saccharopolyspora spinosa, Meterhizium anisopliae* ou encore *Beauvaria bassiana.*

7. Procédé selon l'une des revendications 1, 5 et 6, ***caractérisé en ce que*** l'incorporation dans le support de l'inoculum liquide se fait par aspersion ou par pulvérisation.

8. Procédé selon l'une des revendications 1 à 4, **caractérisé en ce que** l'incorporation dans le support dudit milieu nutritif se fait par aspersion ou par pulvérisation.

9. Procédé selon l'une des revendications 1 à 8, ***caractérisé en ce que*** la stérilisation du support se fait en autoclave, par irradiation ou encore par tyndallisation.

## Patentansprüche

1. Verfahren zum Erhalt eines aus einem Agropellet bestehenden getragenen Feststoffinokulums, aus einer biologisch abbaubaren superabsorbierenden Matrix aus Kunstsoff-Verbund, ein Gemisch aus eiweißhaltigen, an Globulinen und Ballaststoffen reichen Biopolymeren, an Stärke reichen Polysaccharid-Biopolymeren, Hemizellulosen und Pektinen und natürlichen Geweben von Glas-Zellen umfassend, wobei das besagte Agropelletimstande ist, Wasser oder eine wässrige Lösung zu absorbieren, die bis zu 500% seines Gewichts reicht, wobei das besagte Agropellet mit einem Nährmedium vorgefüllt ist, ***dadurch gekennzeichnet, dass*** es darin besteht,
a) in dem Träger aus superabsorbierendem Agropellet das konzentrierte flüssige Nährmedium, das zu dem inokulierenden Stamm passt, mit einem Anteil zwischen 25 Gew.-% und 500 Gew.-% einzuarbeiten, wobei die Konzentration des besagten Nährmediums 2 bis 10 Mal höher ist, als jene eines angepassten flüssigen Nährbodens;
b) den in a) erhaltenen Träger zwischen 40°C und 55°C bis zu einem Feststoffanteil zwischen90 Gew.-% und 95Gew.-%zu dehydrieren;
c) den in b) erhaltenen trockenen Trägerin dichten Behältern zu sterilisieren;
d) den Mikrobenstamm, der zuvor in dem in c) erhaltenen sterilen Träger kultiviert worden ist ,mit einer Mikrobendichte, die zwischen dem 1/500und 1/10,vorzugsweise zwischen 1/200 und 1/100 der durch die Normen für ein flüssiges Inokulum desselben Stammes empfohlenen Menge variiert, einzuarbeiten;
e) den in d) erhaltenen gefüllten Träger durch Wärmeofentrocknung zwischen 35°C und 40°C zu dehydrieren, um ein getragenes Feststoffinokulum mit einem Feststoffanteil zwischen90 Gew.-% und 95Gew.-% zu erhalten;
f) das in Schritt e) erhaltene getragene Feststoffinokulum vor Feuchtigkeit geschützt zu verpacken.

2. Verfahren nach Anspruch 1, ***dadurch gekennzeichnet, dass*** sich das konzentrierte wasserfreie Kulturmedium, das in dem Träger enthalten ist, teilweiseundnach und nachdurch Regenwasser oder Gießwasser auflöst, ohne Gefahr zu laufen, ausgewaschen zu werden.

3. Verfahren nach einem der Ansprüche 1 oder 2, ***dadurch gekennzeichnet, dass*** man das besagte Agropellet zur teilweisen und nach und nach stattfindenden Auflösung des besagten Kulturmediums rehydriert, um das Wachstum zu fördern, gefolgt von der fortgesetzten Vermehrung der neubelebten Mikroorganismen, auch dann, wenn das besagte Agropellet vergraben ist.

4. Verfahren nach einem der Ansprüche 1 bis 3, ***dadurch gekennzeichnet, dass*** sich das besagte Agropellet durch Quellenmittels Absorption von Wasser odereiner wässrigen Lösung und durch Zurückziehen mittels erneuter Abgabe dieser besagten Flüssigkeiten, ohne zu zerfallen, im Volumen verändert.

5. Verfahren nach einem der Ansprüche 1 bis 4, ***dadurch gekennzeichnet, dass*** die Mikroorganismen aus den Mykorrhizapilzen und den Rhizobakterien ausgewählt werden.

6. Verfahren nach Anspruch 5, ***dadurch gekennzeichnet, dass*** die Mikroorganismen aus *Azotobacter* spp., *Acidovorax facilis, Flavobacterium* spp., *Pseudomonas* spp., *Rhodococcus rhodochrous, Bacillus subtilis, Bacillus chitinoporus, Bacillus laterosporus, Bacillus thuringiensis, Saccharopolyspora spinosa, Meterhizium anisopliae* oder aber *Beauvaria* bassianaausgewählt werden.

7. Verfahren nach einem der Ansprüche 1, 5 und 6, ***dadurch gekennzeichnet, dass*** die Einarbeitung des flüssigen Inokulums in den Träger durch Aspersion oder durch Zerstäuben erfolgt.

8. Verfahren nach einem der Ansprüche 1 bis 4, ***dadurch gekennzeichnet, dass*** die Einarbeitung des besagten Kulturmediums in den Träger durch Aspersion oder durch Zerstäuben erfolgt.

9. Verfahren nach einem der Ansprüche 1 bis 8, ***dadurch gekennzeichnet, dass*** die Sterilisierung des Trägers durch Autoklav, durch Bestrahlen oder aber durch Tyndallisation erfolgt.

## Claims

1. Production method for a solid inoculum carrier made of a superabsorbent biodegradable plastic composite matrix agro-pellet comprising a mixture of protein biopolymers rich in globulins and fibres, of a polysaccharide biopolymers rich in starch, hemicellulose and pectin and of a natural hyaline cell tissue, said agro-pellet is capable of absorbing water or an aqueous solution up to 500% of its own weight, said agro-pellet being pre-loaded with a nutritive medium, ***characterised in that*** it consists in:
a) incorporating the concentrated liquid nutritive medium adapted to the strain to inoculate into the superabsorbent agro-pellet, at a level between 25% and 500% of its weight, said nutritive medium being in a concentration from 2 to 10 times greater than that of an adapted liquid culture medium;
b) dehydrating the support obtained in a) between 40°C and 55°C until the dry matter content reaches a level between 90% and 95% by weight;
c) sterilising the dry support obtained in b) in air-tight containers;
d) incorporating the previously cultured microbial strain into the sterile support obtained in c) with a microbial density varying between 1/500^{th} and 1/10^{th}, but preferentially a microbial density of between 1/200^{th} and 1/100^{th} of the quantity recommended by the standards for a liquid inoculation of the same strain;
e) dehydrating the support loaded in d) by a dry heat at a temperature between 35°C and 40°C to obtain a solid inoculum carrier having a dry matter content between 90% and 95% by weight;
f) packaging the solid inoculum carrier obtained in e) to avoid contact with humidity.

2. A method according to the claim 1, ***characterised in that*** the concentrated anhydrous nutritive medium contained in the support is dissolved partially and progressively by rain or irrigation water with no risk of leaching.

3. A method according to one of the claims 1 or 2, ***characterised in that*** the rehydration of said agro-pellet and the partial and progressive dissolution of said nutritional medium promotes the sustained increase in continuous multiplication of the regrown microorganisms, including when said agro-pellet is interred.

4. A method according to one of claims 1 to 3, ***characterised in that*** said agro-pellet varies in volume, swelling via water or aqueous solution absorption and retracting by releasing these said liquids without disintegrating.

5. A method to one of claims 1 to 4, ***characterised in that*** the microorganisms are chosen from mycorrhizal fungus and rhizobacteria.

6. A method according to the claim 5, ***characterised in that*** microorganisms are chosen from *Azotobacter* spp., *Acidovorax facilis, Flavobacterium* spp., *Pseudomonas* spp., *Rhodococcus rhodochrous, Bacillus subtilis, Bacillus chitinoporus, Bacillus laterosporus, Bacillus thuringiensis, Saccharopolyspora spinosa, Meterhizium anisopliae* or *Beauvaria bassiana*.

7. A method according to one of claims 1, 5 and 6, ***characterised in that*** the liquid inoculum is incorporated into the support by sprinkling or spraying.

8. A method according to one of claims 1 to 4, ***characterised in that*** the incorporation of said nutritive liquid medium into the support is performed by sprinkling or by spraying.

9. A method according to one of claims 1 to 8, ***characterised in that*** the support is sterilised in an autoclave, by irradiation, or by tyndallisation.
